# EUROPÄISCHE PATENTSCHRIFT

(11) **EP 3 921 570 B1**
(45) Veröffentlichungstag und Bekanntmachung des Hinweises auf die Patenterteilung: **02.04.2025**
(21) Anmeldenummer: 20702666.7
(22) Anmeldetag: 06.02.2020
(51) Int. Cl.: F16M 11/04, F16M 11/08, F16M 13/02

(54) **FALLSICHERUNGSVORRICHTUNG FÜR EIN MEDIZINISCHES TRAGSYSTEM, TRAGSYSTEM FÜR EIN MEDIZINISCHES GERÄT, VERFAHREN ZUR ANBRINGUNG EINER FALLSICHERUNGSVORRICHTUNG AN EINEM TRAGSYSTEM**
SECURITY CATCH FOR A MEDICAL SUPPORT SYSTEM, SUPPORT SYSTEM FOR A MEDICAL DEVICE, METHOD FOR APPLYING A SECURITY CATCH ON A SUPPORT SYSTEM
DISPOSITIF DE PROTECTION CONTRE LES CHUTES POUR UN SYSTÈME DE SUPPORT MÉDICAL, SYSTÈME DE TRANSPORT POUR UN APPAREIL MÉDICAL, PROCÉDÉ DE FIXATION D'UN DISPOSITIF DE PROTECTION CONTRE LES CHUTES SUR UN SYSTÈME DE SUPPORT

(30) Priorität: 06.02.2019 EP 19155829
(43) Veröffentlichungstag der Anmeldung: 15.12.2021
(73) Patentinhaber: Ondal Medical Systems GmbH, 36088 Hünfeld (DE)
(72) Erfinder: MURATIDI, Georg, 36280 Oberaula (DE); PERPLIES, Stefan, 36088 Hünfeld (DE)
(74) Vertreter: Graf von Stosch Patentanwaltsgesellschaft mbH
(86) Internationale Anmeldenummer: PCT/EP2020/053050
(87) Internationale Veröffentlichungsnummer: WO 2020/161268

(56) Entgegenhaltungen:
- CN-U- 202 521 191
- GB-A- 2 475 298
- KR-A- 20120 115 830
- US-A1- 2009 173 856

## Beschreibung

Die vorliegende Erfindung betrifft eine Fallsicherungsvorrichtung für ein Tragsystem zum Halten wenigstens eines medizinischen Geräts, ein Tragsystem für ein medizinisches Gerät sowie ein Verfahren zur Anbringung einer Fallsicherungsvorrichtung an einem Tragsystem für ein medizinisches Gerät.

Tragsysteme werden beispielsweise in Operationssälen zum örtlichen verlagerbaren oder örtlich festgelegten Halten einer medizintechnischen Einrichtung genutzt. Beispielsweise kann dazu ein Tragarm beweglich mittels einer Lageranordnung an einer Decke, einer Wand oder einem Boden verankert sein. Auch eine Befestigung auf einem Gestell mit Rollen ist möglich. Ein Tragsystem mit Tragarmen kann auch als Tragarmsystem bezeichnet werden und ein Tragsystem, welches zum Halten medizinischer Geräte ausgebildet ist, kann auch als medizinisches Tragsystem bezeichnet werden. Tragsysteme weisen üblicherweise wenigstens einen beweglichen Tragarm zum Halten des medizinischen Geräts, welches auch als medizintechnisches Gerät bezeichnet werden kann, beispielsweise einer OP-Lampe, OP-Besteck oder zahnmedizinische Bohrer, auf. Ebenso können jeweilige Tragarmsysteme auch wenigstens eine Bremsvorrichtung umfassen, welche dazu ausgebildet ist, eine Stellung des Tragarms und damit des medizinischen Geräts festzulegen und/oder jeweilige Bewegungen des Tragarms und damit des medizinischen Geräts zu Hemmen. Das örtliche Verlagern medizinischer Geräte ist bei vielen medizinischen Prozeduren essentiell, um dem medizinischen Personal die Arbeit zu erleichtern oder überhaupt zu ermöglichen.

Durch das häufige Bewegen der einzelnen Teile des Tragsystems und/oder durch das Anbringen unterschiedlicher Geräte wird ein solches System und dessen Teile stark belastet. Durch Marktbeobachtungen konnte dabei festgestellt werden, dass der Umgang mit solchen Tragarmsystemen bei Anwendern robuster wird. Insbesondere werden Gelenke durch die von den Anwendern eingeleiteten dynamischen Kräfte stärker beansprucht und diese Belastungen von medizinischen Tragarmsystemen durch Bedienpersonal dürften sich sogar noch weiter erhöhen. Häufigere und/oder stärkere Bewegungen können zu strukturellen Ermüdungserscheinungen führen, in deren Folge es zu Brüchen an besonders belasteten Elementen oder Kraftangriffspunkten kommen kann. Auch häufig wechselnde Gewichte und/oder ein hohes Gewicht jeweiliger medizinischer Geräte belasten die strukturelle Integrität von Tragsystems über deren Lebenszeit zunehmend. Zudem kann es im Rahmen zeitintensiver Notoperationen oder auch im regulären Betrieb bei verkürzten Krankenhausprozessen zu Fehlgebrauch und hierdurch zu Belastungen von medizinischen Tragsystem kommen, beispielsweise durch ein unzulässiges Abstützen oder Aufsteigen einer Person auf Teile des Tragsystems. Hierauf könnte mit mechanisch robusteren Bauweisen reagiert werden.

Trotzdem können bei dauerhaft unsachgemäßem und robustem Gebrauch Teile eines Tragsystems brechen, insbesondere an maximal beanspruchten Kraftangriffspunkten. Dennoch dürfen abgebrochene Bauteile Patienten oder medizinisches Personal keinesfalls gefährden. Aus diesem Grund kann eine Fallsicherung bei medizinischen Tragsystemen vorgesehen werden, welches auch bei einem Bruch ein Herabfallen von Bauteilen zuverlässig verhindert und so eine sichere Benutzung des Tragsystems auch über einen langen Benutzungszeitraum, sogar bei unsachgemäßem Gebrauch, ermöglicht. Ein Herabfallen von abgebrochenen Bauteilen und ein Heraufschnellen des verbliebenen Tragarms kann so verhindert werden. Gleichzeitig ist und soll es für die Anwender ohne weiteres erkennbar bleiben, dass ein Austausch oder eine Reparatur des Systems nach Gerätebruch notwendig ist. Eine fortgesetzte Benutzung eines derartig beschädigten Systems ist ohnedies nahezu ausgeschlossen.

Eine solche als Fangvorrichtung bezeichnete Fallsicherung ist beispielsweise aus der DE 100 51 898 B4 bekannt. Diesbezüglich ist es vorgesehen, eine Sicherungsschlinge um einen Bolzen zu schlingen, wobei die Sicherungsschlinge jeweilige Verbreiterungen an ihren Enden aufweist und mit diesen Enden jeweilige in einem Rückhaltekörper ausgebildete Öffnungen durchgreift. Dieser Rückhaltekörper ist mit einem Hauptkörper lösbar verbindbar. Diese Fallsicherung benötigt jedoch zahlreiche Bauteile und ist dementsprechend aufwendig herzustellen, schwierig zu montieren, ausgesprochen schwer und insgesamt teuer in der Herstellung. Zudem müssen für die verschiedenen Teile und deren Anbringung ein entsprechender Bauraum und Befestigungsmöglichkeiten vorgesehen werden.

Weiterhin ist aus der GB 2 476 298 A eine Haltevorrichtung für ein Medizingerät bekannt, wobei ein extern geführtes Sicherheitskabel mittels Schrauben und Windungen zwischen zwei Komponenten der Haltevorrichtung vorgesehen ist.

Aufgabe der vorliegenden Erfindung ist es, eine einfache und kostengünstige Fallsicherung für medizinische Tragsystem bereitzustellen.

Diese Aufgabe wird erfindungsgemäß durch die Gegenstände der unabhängigen Patentansprüche gelöst. Vorteilhafte Ausgestaltungen mit zweckmäßigen Weiterbildungen der Erfindung sind in den jeweiligen Unteransprüchen angegeben, wobei vorteilhafte Ausgestaltungen eines Aspekts als vorteilhafte Ausgestaltungen jeweiliger anderer Aspekte und umgekehrt anzusehen sind.

Ein erster Aspekt der Erfindung betrifft eine Fallsicherungsvorrichtung für ein Tragsystem zum vorzugsweise bewegbaren Halten wenigstens eines medizinischen Geräts. Bei dem medizinischen Gerät kann es sich beispielsweise um eine Ablage für OP-Besteck, medizinische Bohrer, Überwachungsvorrichtungen und Monitore oder eine OP-Lampe handeln. Das Tragsystem kann beispielsweise mehrere Tragarme aufweisen und beispielsweise selbsttragend oder an einer Wand oder Decke montiert sein. Die Tragarme können beweglich aneinander gelagert sind, um die Position des medizinischen Geräts weitestgehend frei einzustellen. Das Tragsystem und/oder die Fallsicherungsvorrichtung weist wenigstens eine Tragvorrichtung auf, mittels welcher das medizinische Gerät gehalten ist. Die Tragvorrichtung kann also jeweilige Bauteile aufweisen, mittels welcher das medizinische Gerät vom Boden beabstandet gehalten werden kann.

Bewegbares Halten stellt typischerweise gerade keine feste Verschraubung von Komponenten dar.

Die Tragvorrichtung weist eine kritische Stelle auf. Die kritische Stelle ist beispielsweise als eine Stelle oder ein Bereich definiert, an welchem die Tragvorrichtung, beispielsweise bei fortgesetzter robuster oder unsachgemäßer Benutzung und/oder unüblicher Überlastung, besonders bruchgefährdet ist. Beispielsweise kann die kritische Stelle oder der kritische Punkt die oder eine der höchstbelastete(n) Stelle(n) bzw. der höchstbelastete oder einer der höchstbelasteten Punkt(e) bei ordnungsgemäßem und/oder unsachgemäßem Gebrauch der Tragvorrichtung sein, d.h. der/die Angriffspunkt(e) in der Tragevorrichtung, in denen Kraftspitzen auftreten und daher Ermüdungsbrüche mit höherer Wahrscheinlichkeit auftreten können. Die kritische Stelle(n) kann/können beispielsweise durch Strukturberechnungen, Experimente, und/oder Erfahrungswerte identifiziert werden oder aber auch durch die Bereitstellung einer Sollbruchstelle in der Struktur der Tragvorrichtung konstruktiv vorgegeben werden.

Die Fallsicherungsvorrichtung dient der Vermeidung des Sollbruchrisikos von Bauteilen der Tragvorrichtung. Sie weist ein längliches Sicherungsteil auf. Mittels dieses Sicherungsteils können jeweilige Bereiche der Tragvorrichtung, welche durch einen Bruch an einer kritischen Stelle getrennt werden, zusätzlich miteinander verbunden sein. Dadurch kann ein Herabfallen eines der beiden Bruchteile auch bei einem Bruch an einer kritischen Stelle verhindert werden. Zu diesem Zweck ist das Sicherungsteil vorzugsweise beidseitig der kritischen Stelle jeweils mit der Tragvorrichtung verbunden bzw. verbindbar. Das Sicherungsteil ist vorzugsweise so ausgelegt, dass es ein an einer kritischen Stelle abgebrochenes Teil mit einem daran befestigten medizinischen Gerät auch bei einer gewissen Fallstrecke halten kann. Die Fallstrecke kann durch eine Länge des Sicherungsteils begrenzt sein. Die Fallsicherungsvorrichtung kann somit helfen, eine kritische Stelle und/oder eine in der Herstellung eingeführte Sollbruchstelle abzusichern.

Weiterhin weist die Tragvorrichtung eine erste Durchgangsöffnung in einem ersten Bereich auf, welcher von einer kritischen Stelle beabstandet ist. Weiterhin weist die Tragvorrichtung eine zweite Durchgangsöffnung in einem zweiten von einer kritischen Stelle beabstandeten Bereich auf. Der zweite Bereich ist bezüglich der kritischen Stelle dem ersten Bereich gegenüberliegend angeordnet. Eine kritische Stelle kann also als Trennung zwischen dem ersten und dem zweiten Bereich angesehen werden. Insbesondere kann eine kritische Stelle als durchgängige Fläche durch die Tragvorrichtung angesehen werden, welche diese in zwei Teile teilt, insbesondere bei einem Bruch der Tragvorrichtung an einer kritischen Stelle. In dem einen Teil ist somit der erste Bereich angeordnet und in dem anderen Teil der zweite Bereich. Die jeweiligen Bereiche können dabei auch als Endbereiche der Tragvorrichtung ausgebildet sein und/oder einem jeweiligen Element der Tragvorrichtung entsprechen, beispielsweise einem Tragelement wie einem Tragarm. Vorliegend weist das Tragsystem ein erstes Tragelement, welches die erste Durchgangsöffnung aufweist, und ein zweites Tragelement, welches die zweite Durchgangsöffnung aufweist, auf. Vorzugsweise sind die jeweiligen Durchgangsöffnungen von einer kritischen Stelle soweit beabstandet, dass die Wandungen der Durchgangsöffnungen bei einem Bruch an einer kritischen Stelle nicht vom Bruch betroffen sind bzw. deren Dicke bzw. Stärke so groß ist, dass an den Durchgangsöffnungen das abgebrochene Teil mit dem Sicherungsteil gehalten werden kann.

Das Sicherungsteil ist durch beide Durchgangsöffnungen geführt. Die erste Durchgangsöffnung ist dabei an einem ersten Tragelement und die zweite Durchgangsöffnung ist dabei an einem zweiten Tragelement vorgesehen, wobei die Durchgangsöffnungen in einer einen Innenraum begrenzenden Wandung der Tragvorrichtung vorgesehen sind. Das Sicherungsteil wird zwischen den Durchgangsöffnungen und an einer Ausnehmung des ersten Tragelements in den Innenraum geführt. Zudem weist die Fallsicherungsvorrichtung ein erstes an einem ersten Ende des Sicherungsteils befestigtes Blockierelement und ein zweites an einem zweiten, dem ersten Ende gegenüberliegendem Ende des Sicherungsteils befestigtes Blockierelement auf. Die Blockierelemente sind dabei derart angeordnet, dass ein Herausgleiten des Sicherungsteils aus den beiden Durchgangsöffnungen blockiert ist. Entsprechend kann so das Sicherungsteil an den beiden Durchgangsöffnungen befestigt sein. Somit sind die zwei Bruchteile bei einem Bruch der kritischen Stelle weiter mittels des an den Durchgangsöffnungen gehaltenen Sicherungsteils miteinander verbunden. Dadurch kann ein Herabstürzen des abgebrochenen Bauteils, bzw. eines Tragarms, zuverlässig verhindert werden.

Die Fallsicherungsvorrichtung benötigt nur wenige Bauteile. Insbesondere kann auf jeweilige Befestigungsbolzen und/oder Schlingen verzichtet werden. Dadurch ist die Fallsicherungsvorrichtung leicht und kostengünstig. Zudem benötigt die Fallsicherungsvorrichtung beispielsweise lediglich Bauraum zum Führen des Sicherungsteils von einer Durchgangsöffnung zur anderen und keinen zusätzlichen Bauraum zur Verankerung, wodurch dieser Bauraum für andere Bauteile zur Verfügung steht. Die Blockierelemente können beispielsweise außenseitig an der Tragvorrichtung angeordnet sein. Auch die Montage der Fallsicherungsvorrichtung ist sehr einfach, da beispielsweise erst ein freies Ende durch die beiden Durchgangsöffnungen gefädelt werden kann und danach ein Blockierelement an dem freien Ende befestigt werden kann. Zudem kann so einfach eine spielfreie Montage erreicht werden, da die nachträgliche Anbringung des Blockierelements eine einfache Anpassung an den tatsächlichen Abstand zwischen den beiden Durchgangsöffnungen ermöglicht. Das Sicherungsteil kann auch unter Spannung stehend an der Tragvorrichtung bzw. den beiden Durchgangsöffnungen festgelegt werden. Ein geringes Spiel minimiert eine Fallstrecke bei einem Bruch der Tragvorrichtung und damit zusätzliche dynamische Falllasten, für die das Sicherungsteil und jeweilige die Durchgangsöffnungen bildende Wandungen ausgelegt werden müssen. Eine überstehende Länge des Sicherungsteils kann nach Befestigung beispielsweise abgeschnitten werden. Das Sicherungsteil kann so auch kostengünstig mit großen Fertigungstoleranzen oder als Endlosteil, welches bedarfsgerecht zugeschnitten wird, zur Verfügung gestellt werden.

In weiterer vorteilhafter Ausgestaltung der Fallsicherungsvorrichtung ist die Fallsicherungsvorrichtung so ausgebildet, dass das Sicherungsteil bei Gebrauch des Tragsystems, insbesondere vor einem Bruch der kritischen Stelle, lastfrei ist. Lastfrei kann beispielsweise bedeuten, dass das Sicherungsteil keinen oder nur einen vernachlässigbaren Beitrag zum Aufnehmen einer Last des gehaltenen medizinischen Geräts leistet. Beispielsweise kann auch keiner der beiden Bereiche des Tragsystems von dem Sicherungsteil vor dem Bruch der kritischen Stelle gehalten sein. Beispielsweise kann ein Mittenbereich des Sicherungsteil eine Länge aufweisen, welche größer ist, als eine kürzeste Verbindung zwischen den beiden Durchgangsöffnungen. Das Sicherungsteil kann beispielsweise teilweise herabhängend und/oder spannungsfrei zwischen den Durchgangsöffnungen angeordnet sein. Erst nach Bruch der kritischen Stelle nimmt das Sicherungsteil das Gewicht des medizinischen Geräts und jeweiliger abgebrochener und daran hängender Teilbereiche des Tragsystems auf. Dadurch, dass das Sicherungsteil lastfrei ist, wird dieses keinen Belastungen im ordnungsgemäßen Gebrauch und/oder bei unsachgemäßem Gebrauch des Tragarmsystems ausgesetzt, sofern die kritische Stelle noch nicht gebrochen ist. Das lastfreie Sicherungsteil hält vor dem Bruch der kritischen Stelle beispielsweise nicht zwei Teile des Tragsystems zusammen oder befestigt diese aneinander. Dadurch kann eine Materialermüdung und/oder ein Verschleiß des Sicherungsteils vor dem Bruch der kritischen Stelle vermieden werden.

Vorzugsweise hat das Sicherungsteil entlang seiner länglichen Erstreckung einen Durchmesser, welcher kleiner als jeweilige Durchmesser der Durchgangsöffnungen ist. Vorzugsweise sind die Blockierelemente auf voneinander abgewandten Seiten angeordnet, wobei einander zugewandte Seiten durch die beiden Seiten definiert sind, von denen sich das Sicherungsteil von einer Durchgangsöffnung zur anderen Durchgangsöffnung erstreckt.

Die Tragvorrichtung kann auch mehrere kritische Stellen aufweisen. In diesem Fall kann pro kritischer Stelle ein Sicherungsteil mit jeweils zwei zugehörigen, beidseitig der kritischen Stelle angeordneten Durchgangsöffnungen vorgesehen sein. Dadurch kann das Sicherungsteil und jeweilige Wandungen an den Lastfall bei Bruch jeder einzelnen kritischen Stelle angepasst ausgelegt werden. Zudem kann so die Fallstrecke besonders gering sein. Es kann aber auch vorgesehen werden, dass ein gemeinsames Sicherungsteil und ein zugehörige Paar von Durchgangsöffnungen für mehrere kritische Stellen vorgesehen werden. In diesem Fall können die mehreren kritische Stellen gesamthaft als eine gemeinsame kritische Stelle gesehen werden, zu denen beidseitig jeweils eine Durchgangsöffnung vorzusehen ist. Hierdurch kann die Anzahl der Teile für die Fallsicherung besonders gering sein. Das Sicherungsteil und jeweilige die Durchgangsöffnungen bildende Wandungen sollten dabei für den Bruch derjenigen der mehreren kritische Stellen ausgelegt werden, bei dem die größte Belastung zu erwarten ist.

In weiterer vorteilhafter Ausgestaltung der Fallsicherungsvorrichtung ist es vorgesehen, dass die kritische Stelle ein Gelenk der Tragvorrichtung, eine Lagerung zwischen zwei Teilen der Tragvorrichtung, an welcher diese miteinander beweglich verbunden sind, ein Winkelstück der Tragvorrichtung, und/oder eine Fügestelle, insbesondere eine Schweißnaht, der Tragvorrichtung ist. Dadurch können diese teuren und/oder schweren Komponenten gegebenenfalls baulich einfacher und mit reduzierten Kosten ausgelegt werden, da Personen bei einem Bruch der Tragvorrichtung jedenfalls durch die Fallsicherungsvorrichtung geschützt sind. So kann zudem gegebenenfalls auf eine aufwendige Strukturberechnung und insbesondere Ermüdungsberechnung verzichtet werden. Alternativ oder zusätzlich kann die kritische Stelle ein strukturell schwacher oder schwächster Bereich der Tragvorrichtung sein. Alternativ oder zusätzlich kann kritische Stellen die bei sachgemäßem und/oder unsachgemäßem Gebrauch ein oder mehr bruchgefährdete Punkte in der Tragarmvorrichtung, die Kraftspitzen ausgesetzt sind sein. Auch ein Verschleiß und/oder eine Ermüdung kann bei der Ermittlung von potentiell kritischen Stellen a priori berücksichtigt werden. Beispielsweise kann als kritische Stelle ein Bereich eines Tragelements angesehen werden, welcher eine dünnere Wandung hat als andere Bereiche und/oder Tragelemente. Auch eine konstruktiv vorgegebene Sollbruchstelle kann so als kritische Stelle vorgesehen sein. Dabei können auch Lastberechnungen und Ermüdungsberechnungen berücksichtigt werden, um sinnvolle Stellen zu bestimmen, an denen eine Fallsicherung vorgesehen werden soll.

In weiterer vorteilhafter Ausgestaltung der Fallsicherungsvorrichtung ist es vorgesehen, dass das längliche Sicherungsteil als biegeschlaffes Element ausgebildet ist. Dadurch kann das Sicherungsteil besonders einfach durch die Durchgangsöffnungen geführt werden. Auch falls die Durchgangsöffnungen nicht auf einer Linie liegen, sondern beispielsweise über Eck verbunden werden müssen, ist die Montage so vereinfacht. Zudem kann sich das biegeschlaffe Element auch in seiner Form anpassen, falls die beiden Durchgangsöffnungen zueinander bewegt werden. Dies ist beispielsweise häufig der Fall, wenn das Sicherungsteil eine Lagerung oder ein Gelenk als kritische Stelle absichert. Ein einfaches und kostengünstiges Beispiel für ein biegeschlaffes Element ist ein Seil, welches hier dann auch als Fangseil bezeichnet werden kann. Das Seil kann beispielsweise ein Metallseil, wie ein Drahtseil sein, ein Kunststoffseil oder aus natürlichen Fasern gebildet sein. Alternativ zu einem Seil kann beispielsweise auch ein Metalldraht oder ein flexibles Kunststoffsteil vorgesehen werden, wie ein Kunststoffsteg.

In weiterer vorteilhafter Ausgestaltung der Fallsicherungsvorrichtung ist es vorgesehen, dass das erste Blockierelement fest mit dem Sicherungsteil verbunden ist und/oder einteilig mit diesem ausgebildet ist. So kann das Sicherungsteil besonders einfach montiert werden, da ein versehentliches Herausziehen des Sicherungsteils aus einer der beiden Durchgangsöffnungen beim Einfädeln in die andere Durchgangsöffnung durch das bereits befestigte Blockierelement verhindert wird. Zudem ist es so beispielsweise höchstens notwendig, eines der Blockierelemente beim Aufbau bzw. Zusammenbau des Tragsystems bzw. der Fallsicherungsvorrichtung zu befestigten, wodurch Aufbau und Montage schnell und einfach sind. Das feste Blockierelemente kann beispielsweise angespritzt sein, angegossen, angelötet oder auch angeklebt. Beispielsweise kann das feste Blockierelement als Kunststoffnippel an dem als Drahtseil ausgebildeten Sicherungsteil angespritzt sein. Eine solche Bauweise ist besonders kostengünstig herstellbar.

In weiterer vorteilhafter Ausgestaltung der Fallsicherungsvorrichtung ist es vorgesehen, dass das zweite Blockierelement dazu ausgebildet ist, erst nach dem Durchführen des Sicherungsteils durch die beiden Durchgangsöffnungen an dem Sicherungsteil befestigt zu werden. Dadurch steht zunächst ein freies Ende des Sicherungsteils zur Verfügung, welches einfach durch die beiden Durchgangsöffnungen nacheinander eingeschoben werden kann. Das zweite Blockierelement kann nach Durchführen des Sicherungsteils durch die Durchgangsöffnungen dauerhaft, also fest, oder auch lösbar, an dem freien Ende befestigt werden, um das Sicherungsteil beidseitig der kritischen Stelle und damit potenziellen Bruchstelle an der Tragvorrichtung zu befestigten. Ein einfaches Beispiel für eine lösbare Befestigung ist ein Anschrauben des zweiten Blockierelements. Durch ein lösbares Befestigen kann die Fallsicherungsvorrichtung und damit auch das Tragsystem einfach und zerstörungsfrei wieder demontiert werden. Alternativ kann das zweite Befestigungselement auch dauerhaft bzw. nicht zerstörungsfrei lösbar befestigt werden. Beispielsweise kann das zweite Befestigungselement aufgeklebt, angelötet, angeschweißt, ancrimpen oder aufgepresst werden. Insbesondere ein Aufpressen kann einfach manuell erfolgen, beispielsweise mittels einer Zange. Dadurch kann die Fallsicherungsvorrichtung leicht vor Ort nach Zusammenbau des Tragsystems angebracht werden. Durch die dauerhafte Befestigung kann insbesondere ein ungewolltes Lösen der Fallsicherung verhindert werden.

Jeweilige Blockierelemente können auch durch das Sicherungsteil selber gebildet sein, wodurch die Teilezahl besonders gering sein kann. Beispielsweise bei einem seilartigen Sicherungsteil kann das Blockierelement durch einen Knoten in dem Seil gebildet werden. Das Sicherungsteil kann aber beispielsweise auch bewegliche Teile am Ende aufweisen, welche zum Bereitstellen der Blockierung verstellt werden können.

In weiterer vorteilhafter Ausgestaltung der Fallsicherungsvorrichtung ist es vorgesehen, dass die jeweiligen Blockierelemente so gestaltet sind, dass diese nicht durch die jeweilige zugeordnete Durchgangsöffnung gleiten können. Beispielsweise können die Blockierelemente nach Art einer Hülse oder eines Nippels über einen Endbereich eines als Fangseil ausgebildeten Sicherungsteils gestülpt sein. Vorzugweise ist die Form und/oder Größe der jeweiligen Blockierelemente so, dass diese nicht durch die Durchgangsöffnungen passen. Beispielsweise kann eine Hülse und/oder ein Nippel einen Durchmesser aufweisen, welcher größer als ein Durchmesser der jeweiligen Durchgangsöffnungen ist. Die Blockierelemente können auch als Aufdickungen des Sicherungsteils, insbesondere als aufgedickte Enden eines Fangseils, ausgebildet sein.

In der Ausgestaltung der Fallsicherungsvorrichtung ist es vorgesehen, dass die Tragvorrichtung einen Innenraum begrenzt und wenigstens eine der Durchgangsöffnungen vom Innenraum nach außen, also außerhalb des Innenraums, beispielsweise zu einer Außenseite der Tragvorrichtung, führt. Dadurch kann das Sicherungsteil zu einer Außenseite der Tragvorrichtung geführt sein, sodass es für die Anbringung des Blockierelements insbesondere am freien Ende bei der Montage zugänglich ist. Durch wenigstens ein außenseitig angebrachtes Teil, wie beispielsweise ein Bereich des Sicherungsteils und/oder eines der Blockierelemente, kann zudem leicht optisch kontrolliert werden, dass tatsächlich an dem Tragsystem die Fangsicherung angebracht wurde. Bevorzugt führen beide Durchgangsöffnungen von einem Innenraum nach außen, sodass die Montage des Sicherungsteils besonders einfach von außen möglich ist. Die Durchgangsöffnungen sind in einer den Innenraum begrenzenden Wandung bzw. Wandungen vorgesehen. Zudem benötigen so jeweilige Blockierelemente keinen Bauraum innerhalb der Tragvorrichtung. Dieser steht dann beispielsweise für Leitungen zur Verfügung. Das Sicherungsteil kann dagegen wenigstens teilweise innenseitig geführt sein, um es vor Beschädigungen zu schützen. Gerade ein biegeschlaffes Sicherungsteil kann sich dabei in der Form leicht an einen zur Verfügung stehenden inneren Durchgang anpassen. Der Innenraum kann komplett geschlossen sein. Alternativ kann der Innenraum auch einseitig offen sein oder nach Art einer Durchgangsöffnung ausgebildet sein. Beispielsweise weist die Tragvorrichtung häufig eine oder mehrere Hohlprofile auf, welche jeweilige Tragarme und den Innenraum bilden. Der innere Durchgang dieser Hohlprofile kann dabei mit einer Außenseite der Hohlprofile durch eine oder jeweils beide Durchgangsöffnungen verbunden sein.

Durch die Bauweise der Fallsicherungsvorrichtung kann beispielsweise lediglich ein Mittelteil des Sicherungsteils in dem Innenraum der Tragvorrichtung angeordnet sein und die beiden Blockierelemente außerhalb, oder umgekehrt. Im Gegensatz zu einem Rückhaltekörper ist dabei der Innenraum nicht nahezu vollständig belegt, sondern lediglich geringfügig verkleinert. Es werden keine Bolzen oder Rückhaltekörper benötigt, die Bauraum im Innenraum belegen, sondern Durchgangsöffnungen, welche tendenziell eher zusätzlichen Bauraum bereitstellen. Dadurch können hier weiterhin beispielsweise Kabel durchgeführt werden, beispielsweise Datenleitungen und/oder eine Stromversorgung für das medizinische Gerät. Zudem blockiert die Fallsicherungsvorrichtung auch keine Montage und/oder Durchführung von Schleifringen an einem Gelenk.

In weiterer vorteilhafter Ausgestaltung der Fallsicherungsvorrichtung ist es vorgesehen, dass wenigstens eines der beiden Blockierelemente außerhalb des Innenraums angeordnet ist. Vorzugsweise sind beide Blockierelemente außerhalb angeordnet. Dadurch kann beispielsweise das üblicherweise schlankere und zudem vorzugsweise biegeschlaffe Sicherungsteil größtenteils innerhalb angeordnet sein, wodurch besonders viel Bauraum im Innenraum für andere Teile zur Verfügung steht, wobei sich das Sicherungsteils geschützt innen an die innere Form anpassen kann.

In weiterer vorteilhafter Ausgestaltung der Fallsicherungsvorrichtung ist es vorgesehen, dass wenigstens ein Mittenbereich des Sicherungsteils innerhalb des Innenraums angeordnet ist. Nur jeweilige Enden mit Blockierelement sind entsprechend außerhalb des Innenraums angeordnet.

Ein zweiter Aspekt der Erfindung betrifft ein Tragsystem zum Halten wenigstens eines medizinischen Geräts. Dieses Tragsystem weist eine Fallsicherungsvorrichtung gemäß dem ersten Aspekt der Erfindung auf. Vorzugsweise ist das Tragsystem zum bewegbaren Halten des medizinischen Geräts ausgebildet. Weiterhin kann das Tragsystem ein oder mehrere Tragarme aufweisen und entsprechend als Tragarmsystem bezeichnet werden. Die sich aus der Fallsicherungsvorrichtung gemäß dem ersten Erfindungsaspekt ergebenden Merkmale und Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

In weiterer vorteilhafter Ausgestaltung des Tragsystems ist es vorgesehen, dass das Tragsystem das erstes Tragelement in Form eines ersten Tragarms, und das zweite Tragelement in Form eines zweiten Tragarms aufweist, welche vorzugsweise mittels eines Gelenks beweglich miteinander verbunden sind. Das Gelenk kann dabei die kritische Stelle bilden, deren Bruch durch die Fallsicherungsvorrichtung abgesichert wird. Das Gelenk kann bevorzugt bspw. im wesentlichen aus Stahl oder Aluminium gefertigt sein Es kann sich um ein Gußteil handeln. Alternativ kann das Gelenkteil auch aus Kunststoff oder einem Kunststoff-VerbundWerkstoff gefertigt sein. So wird ein einfaches, kostengünstiges und leicht zu montierendes Tragsystem bereitgestellt, bei welchem ein Herabfallen eines abgebrochenen Teils bzw. eines Teils aufgrund eines Gelenkbruchs durch das Sicherungsteil und die Blockierelemente verhindert wird.

Ein dritter Aspekt der Erfindung betrifft ein Verfahren zur Anbringung einer Fallsicherungsvorrichtung an einem Tragsystem zum vorzugsweise bewegbaren Halten wenigstens eines medizinischen Geräts. Insbesondere kann es sich also um ein Verfahren zu Montage der Fallsicherungsvorrichtung gemäß dem ersten Aspekt handeln bzw. um ein Verfahren, dass zu dessen Anbringung geeignet ist. Ebenso kann es sich also um ein Verfahren zur Montage der Fallsicherungsvorrichtung, insbesondere der Fallsicherungsvorrichtung, gemäß dem ersten Erfindungsaspekt, an dem Tragsystems gemäß dem zweiten Erfindungsaspekt handeln. Die sich aus der Fallsicherungsvorrichtung gemäß dem ersten Erfindungsaspekt und dem Tragsystem gemäß dem zweiten Erfindungsaspekt ergebenden Merkmale und Vorteile sind den Beschreibungen des ersten bzw. zweiten Erfindungsaspekts zu entnehmen, wobei vorteilhafte Ausgestaltungen des ersten und des zweiten Erfindungsaspekts als vorteilhafte Ausgestaltungen des dritten Erfindungsaspekts und umgekehrt anzusehen sind.

Das Verfahren weist den Schritt des Bereitstellens wenigstens eines länglichen Sicherungsteils mit einem an einem ersten Ende befestigten ersten Blockierelement und einem zweiten, dem ersten Ende gegenüberliegenden freien Ende auf sowie das Bereitstellen wenigstens einer Tragvorrichtung, welche wenigstens eine kritische Stelle, eine erste Durchgangsöffnung in einem ersten von der kritischen Stelle beabstandeten Bereich, und eine zweite Durchgangsöffnung in einem zweiten von der kritischen Stelle beabstandeten Bereich, welcher dem ersten Bereich bezüglich der kritischen Stelle gegenüberliegenden angeordnet ist. In einem weiteren Schritt wird das freie Ende des Sicherungsteils durch die beiden Durchgangsöffnungen geführt, insbesondere nacheinander. Nachfolgend kann das Befestigen eines zweiten Blockierelements an dem freien Ende erfolgen, so dass ein Herausgleiten des Sicherungsteils aus den beiden Durchgangsöffnungen blockiert ist. Die erste Durchgangsöffnung ist dabei an einem ersten Tragelement und die zweite Durchgangsöffnung ist dabei an einem zweiten Tragelement vorgesehen, wobei die Durchgangsöffnungen in einer einen Innenraum begrenzenden Wandung der Tragvorrichtung vorgesehen sind. Das Sicherungsteil wird zwischen den Durchgangsöffnungen und an einer Ausnehmung des ersten Tragelements in den Innenraum geführt. Eine solche Montage ist schnell und einfach. Zudem wird ein Herausgleiten des Sicherungsteils an der Durchgangsöffnung, durch die es zuerst gefädelt wurde, beim Durchführen durch die andere Durchgangsöffnung durch das bereits montierte Blockierelement verhindert.

Vorzugsweise wird das zweite Blockierelement so entlang dem länglichen Sicherungsteil positioniert, dass das Sicherungsteil nahezu spielfrei zwischen den beiden Durchgangsöffnungen positioniert ist und/oder gespannt ist. Spielfrei kann dabei insbesondere bedeuten, dass das Sicherungsteil nicht oder nur geringfügig entlang seiner Längserstreckung beweglich ist. So kann eine Fallstrecke bei Bruch der kritischen Stelle minimiert werden. Ein dann eventuell vorhandener Überstand des Sicherungsteils über das Blockierelement hinaus kann nach der Anbringung des Blockierelements abgetrennt werden.

Auch ein nachträgliches Anbringen bzw. ein Nachrüsten der Fallsicherungsvorrichtung an einem bereits bestehenden Tragsystem ist einfach möglich. Dafür können beispielsweise jeweilige noch nicht vorhandene Durchgangsöffnungen nachträglich eingebracht werden. Beispielsweise können in zwei mittels eines Gelenks verbundene Tragarme jeweils einfach ein Loch als Durchgangsöffnung gebohrt werden, durch die dann das Sicherungsteil geführt wird.

In weiterer vorteilhafter Ausgestaltung des Verfahrens ist es vorgesehen, dass das zweite Blockierelement als Hülse ausgebildet ist, welche zum Befestigen an dem freien Ende auf das Sicherungsteil aufgesteckt und mit diesem verpresst wird. So kann das Sicherungsteil einfach und mit simplem Werkzeug nach dem Durchführen durch die beiden Durchgangsöffnungen an diesen festgelegt werden.

Weitere Merkmale der Erfindung ergeben sich in den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt:
- Fig. 1: in einer schematischen Perspektivansicht ausschnittsweise ein Tragarmsystem für ein medizinisches Gerät mit einem Gelenk;
- Fig. 2: in einer anderen schematischen Perspektivansicht das Tragarmsystem gemäß Fig. 1 mit einer nun daran montierten Fallsicherungsvorrichtung; und
- Fig. 3: in einer schematischen Ansicht, welche zusätzlichen Teile zum Bereitstellen einer Fallsicherung bei dem Tragarmsystem gemäß Fig. 1 benötigt werden.

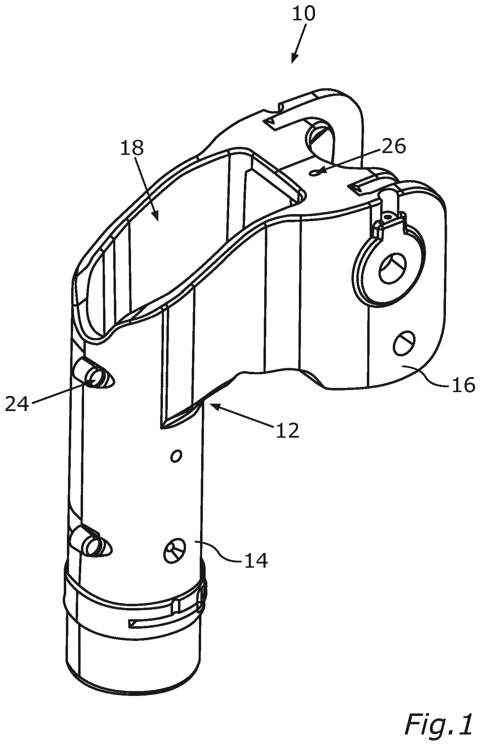

Fig. 1 zeigt in einer schematischen Perspektivansicht ausschnittsweise ein als Tragarmsystem ausgebildetes Tragsystem 10 für ein nicht gezeigtes medizinisches Gerät mit einer Tragvorrichtung, welches zwei mittels eines Gelenks 12 beweglich miteinander verbundene Tragelemente 14 und 16 aufweist. Alternativ kann das Gelenk 12 auch als Winkelstück ausgebildet sein und die beiden Tragelemente 14, 16 starr miteinander verbinden.

Das Tragelement 14 ist dabei zum Halten des medizinischen Geräts an seinem unteren Ende ausgebildet, beispielsweise einer OP-Lampe, einer Operationsvorrichtung und/oder zahnmedizinischer Bohrer. Das Tragelement 14 kann dabei aufgrund des Gelenks 12 gegenüber dem Tragelement 16 rotiert werden, um so die Ausrichtung des medizinischen Geräts einzustellen. Das Tragelement 16 ist an seinem dem Gelenk 12 abgewandten Ende dazu ausgebildet, mit einem Tragarm des Tragsystems 10 verbunden zu werden. Auch diese Verbindung kann ein Gelenk aufweisen, um einen weiteren Freiheitsgrad zur Einstellung der Position des gehaltenen Geräts bereitzustellen.

Das Tragelement 14 ist als Hohlprofil ausgebildet, durch welches jeweilige Kabel zur Stromversorgung des medizinischen Geräts geführt werden können. Das Tragelement 14 und das Tragelement 16 sind mittels eines Schleifkontakts an dem Gelenk 12 miteinander auch elektrisch verbunden. Auch das Tragelement 16 weist einen wenigstens teilweise begrenzten Innenraum 18 auf, in welchem zusätzliche Teile und stromführende Kabel verbaut sein können. Der Innenraum 18 kann dabei durch eine nicht gezeigte Verschlusskappe verschlossen werden, welche beispielsweise an dem Tragelement 16 einschnappt.

Aufgrund häufiger Bewegung des medizinischen Geräts und damit Bewegung der Tragelemente 14, 16 relativ zueinander kann es zu einer unerwartet starken Ermüdung des Gelenks 12 kommen. Auch durch einen häufigen Wechsel des gehaltenen Geräts und/oder Missbrauchslasten kann es zu einer Ermüdung und/oder Überlastung des Gelenks 12 kommen. Auch ein alternativ vorgesehenes Winkelstück kann so überlastet und/oder ermüdet werden. Das Gelenk 12 oder das Winkelstück sind dabei hohen Lasten ausgesetzt und grundsätzlich für einen Bruch anfälliger als beispielsweise die Tragelemente 14, 16 und stellt somit eine kritische Stelle des Tragsystems 10 dar. Gerade eine Ermüdung ist dabei nicht ohne Weiteres erkennbar, sodass es zu einem überraschenden Bruch des Gelenks 12 und Sturz des medizinischen Geräts sowie des Tragelements 14 bei unsachgemäßem Gebrauch und mangelnder Wartung über einen langen Zeitraum kommen kann. Dabei dürfen diese Teile jedoch keinesfalls soweit herabstürzen, dass ein darunter befindlicher Patient oder medizinisches Personal gefährdet werden kann. Zudem kann der Sturz das medizinische Gerät beschädigen und so eine Behandlung gefährden.

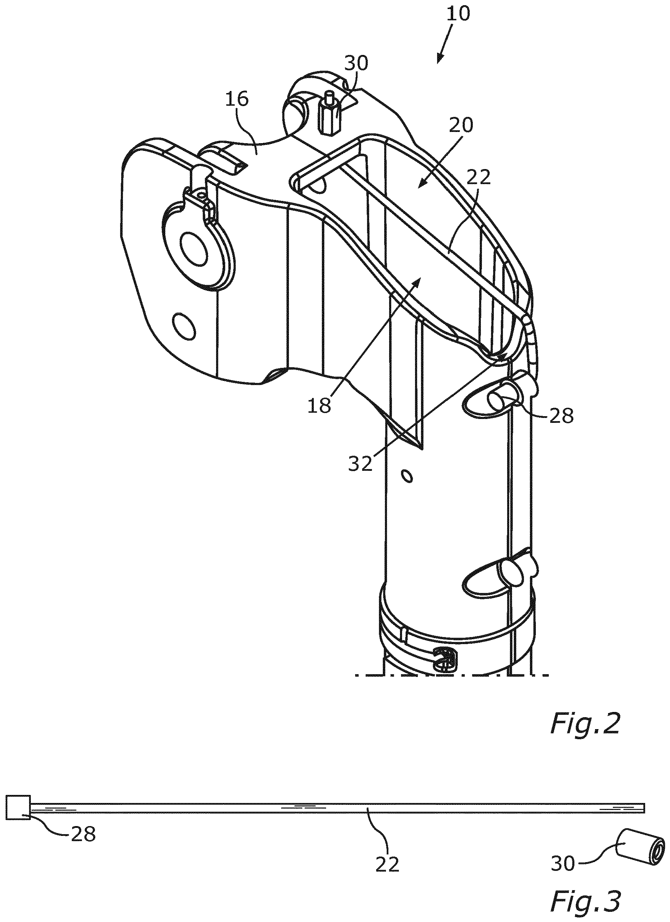

Deshalb weist das Tragsystem 10 eine Fallsicherungsvorrichtung 20, die in der weiteren Perspektivansicht des Tragsystems 10 in Fig. 2 gezeigt ist. Die Fallsicherungsvorrichtung 20 weist dafür ein als Drahtseil 22 ausgebildetes Sicherungsteil 22 auf, mittels welchem die Tragelemente 14, 16 zusätzlich zu dem Gelenk 12 miteinander verbunden sind. Bei einem Bruch des Gelenks 12 ist das Tragelement 14 so weiterhin an dem Tragelement 16 gehalten, sodass weder Menschen noch das medizinische Gerät gefährdet sind.

Dafür weist das Tragelement 14 eine erste Durchgangsöffnung 24 auf und das Tragelement 16 eine zweite Durchgangsöffnung 26. Das Drahtseil 22 ist durch diese beiden Durchgangsöffnungen 24, 26 geführt. An einer der jeweiligen durch das Drahtseil 22 verbundenen Seite der Wandungen, welche die beiden Durchgangsöffnungen 24, 26 bilden, abgewandten Seite sind an jeder der beiden Durchgangsöffnungen 24, 26 ein Blockierelement 28, 30 angeordnet. Durch die beiden Blockierelemente 28, 30 ist das Drahtseil 22 an den beiden Tragelementen 14, 16 an den Durchgangsöffnungen 24, 26 festgelegt und gegen ein Herausgleiten gesichert und hält so diese auch bei einem Bruch des Gelenks 12 noch aneinander.

Bezüglich des Gelenks 12 sind die beiden Durchgangsöffnungen 24, 26 also an voneinander abgewandten Bereichen bzw. in voneinander bezüglich des Gelenks 12 abgewandten Tragelementen 14, 16 beabstandet von dem Gelenk 12 beidseitig zu diesem angeordnet. Durch diese Beabstandung drohen die Durchgangsöffnungen 24, 26 nicht bei einem Bruch des Gelenks 12 mit aufzubrechen und somit das Drahtseil 22 freizugeben.

Wie in Fig. 1 zu erkennen ist, ist die Durchgangsöffnung 26 durch eine Wandung des Tragelements 16 gebohrt, welche den Innenraum 18 wenigstens teilweise begrenzt. Dadurch kann das Drahtseil 22 geschützt durch den Innenraum 18 unter einer Verschlusskappe geführt werden. Das steife Blockierelement 28 ist dagegen außerhalb des Innenraums 18 angeordnet und nimmt dort somit keinen Platz weg. Die Bohrung kann dabei einfach auch noch nachträglich in das Tragelement 16 eingebracht werden.

Wie ebenfalls in Fig. 1 und Fig. 2 zu erkennen ist, ist die Durchgangsöffnung 24 durch eine Bohrung gebildet, welche auch als Verschraubung von zwei Schalen des Hohlprofils des Tragelements 14 dienen könnte. Durch ein straffes, vorgespanntes Befestigen des Drahtseils 22 kann an dieser Stelle auf die Verschraubung verzichtet werden. Das Drahtseil 22 hält dann dort die zwei Schalen zusammen. So kann hier die Fallsicherung nachgerüstet werden, ohne dass eine zusätzliche Bohrung in das Tragelement 14 eingebracht werden muss. Auch das Blockierelement 30 ist außen an dem Tragelement 14 angeordnet. Durch die Anordnung beider Blockierelemente 28, 30 außen kann leicht optisch überprüft werden, ob die Fallsicherungsvorrichtung 20 auch tatsächlich angebracht wurde. Zudem ist eine Montage so besonders einfach.

An dem Tragelement 14 ist das Drahtseil 22 an einer Ausnehmung 32 in den Innenraum geführt. Diese Ausnehmung kann beispielsweise dafür vorgesehen sein, die nicht gezeigte Verschlusskappe abziehen zu können. So muss keine zusätzliche Durchführung für das Drahtseil 22 vorgesehen werden, damit dieses auch bei Nutzung der für eine Verschraubung dienenden Durchgangsöffnung 24 im Innenraum 18 teilweise geführt werden kann.

In Fig. 3 sind schematisch die gegenüber einem herkömmlichen Tragsystem notwendigen Teile zum Bereitstellen einer Fallsicherung gezeigt. Das Blockierelement 28 ist im gezeigten Beispiel ein an dem Drahtseil 22 angespritzter Nippel, beispielsweise ein Kunststoffnippel. Dadurch ist der Durchmesser an diesem Ende des Drahtseils 22 größer als der der Durchgangsöffnungen 24, 26. An dem anderen Ende wird das Drahtseil 22 dagegen ohne Blockierelement zur Verfügung gestellt. Dieses freie Ende kann entsprechend durch die Durchgangsöffnungen 24, 26 zur Anbringung der Fallsicherung gefädelt werden. Anschließend kann das separate Blockierelement 30 an diesem Ende an dem Drahtseil 22 angebracht werden, um dieses an den beiden Tragelementen 14, 16 festzulegen und diese zusätzlich zu dem Gelenk 12 miteinander zu verbinden.

Vorliegend ist das Blockierelement 30 in Fig. 3 beispielhaft als Hülse mit Durchgangsöffnung gezeigt. Diese Hülse kann auf das Drahtseil 22 aufgesteckt werden und zum Festlegen seiner axialen Position an diesem verpresst werden, beispielsweise mittels einer Zange. Die Hülse ist somit leicht zu montieren. Anschließend kann ein Überstand des Drahtseils 22 gekappt werden. Alternativ, wie in Fig. 2 gezeigt, kann das Blockierelement 30 auch aufgeschraubt werden. Dafür kann das Drahtseil 22 einen Gewindebereich aufweisen oder das Blockierelement ein solches Gewinde in das Drahtseil 22 schneiden. Das Sicherungsteil 22 kann in diesem Fall auch vorteilhaft als metallischer Draht ausgebildet sein. Das Blockierelement 30 ist im in Fig. 2 gezeigten Beispiel mit einem Außensechskant für einen einfachen Werkzeugeingriff ausgebildet.

Wie zu erkennen ist, ist die Fallsicherungsvorrichtung 20 einfach zu montieren, benötigt nur wenige zusätzliche Teile, welche zudem leicht und kostengünstig sind, und blockiert kaum Bauraum in dem Innenraum 18 des Tragsystems 10. Die Teile der Fallsicherungsvorrichtung 20 können zudem kostengünstig herstellbare Standardteile sein und müssen nicht spezifisch an die Geometrie der Tragelemente 14, 16 angepasst sein.

### Bezugszeichenliste

- 10: Tragsystem
- 12: Gelenk
- 14: Tragelement
- 16: Tragelement
- 18: Innenraum
- 20: Fallsicherungsvorrichtung
- 22: Sicherungsteils /Drahtseil
- 24: Durchgangsöffnung
- 26: Durchgangsöffnung
- 28: Blockierelement
- 30: Blockierelement
- 32: Ausnehmung

## Patentansprüche

1. Fallsicherungsvorrichtung (20) für ein Tragsystem (10) zum vorzugsweise bewegbaren Halten wenigstens eines medizinischen Geräts,
mit wenigstens einer Tragvorrichtung, welche wenigstens eine kritische Stelle (12) aufweist, und
mit wenigstens einem länglichen Sicherungsteil (22),
wobei die Tragvorrichtung eine erste Durchgangsöffnung (24) in einem ersten von der kritischen Stelle (12) beabstandeten Bereich (14) aufweist und eine zweite Durchgangsöffnung (26) in einem zweiten, dem ersten Bereich bezüglich der kritischen Stelle (12) gegenüberliegenden von der kritischen Stelle beabstandeten Bereich (16) aufweist, wobei das Tragsystem (10) ein erstes Tragelement (14), welches die erste Durchgangsöffnung (24) aufweist, und ein zweites Tragelement (16), welches die zweite Durchgangsöffnung (26) aufweist, aufweist,
wobei das Sicherungsteil (22) durch beide Durchgangsöffnungen (24, 26) geführt ist und die Fallsicherungsvorrichtung (20) ein erstes an einem ersten Ende des Sicherungsteils (22) befestigtes Blockierelement (28) und ein zweites an einem zweiten, dem ersten Ende gegenüberliegendem Ende des Sicherungsteils (22) befestigtes Blockierelement (30) aufweist,
wobei die Blockierelemente (28, 30) derart angeordnet sind, dass ein Herausgleiten des Sicherungsteils (22) aus den beiden Durchgangsöffnungen (24, 26) blockiert ist, und
**dadurch gekennzeichnet, dass** die Durchgangsöffnungen (24, 26) in einer einen Innenraum (18) begrenzenden Wandung der Tragvorrichtung vorgesehen sind, wobei das Sicherungsteil (22) zwischen den Durchgangsöffnungen (24, 26) und an einer Ausnehmung (32) des ersten Tragelements (14) in den Innenraum (18) geführt ist.

2. Fallsicherungsvorrichtung (20) nach Anspruch 1, wobei
die kritische Stelle (12) ein Gelenk (12) der Tragvorrichtung, eine Lagerung zwischen zwei Teilen der Tragvorrichtung, an welcher diese miteinander beweglich verbunden sind, ein Winkelstück der Tragvorrichtung, eine Fügestelle, insbesondere eine Schweißnaht, der Tragvorrichtung, und/oder ein strukturell schwächster Bereich der Tragvorrichtung ist.

3. Fallsicherungsvorrichtung (20) nach Anspruch 1 oder 2, wobei das längliche Sicherungsteil (22) als biegeschlaffes Element ausgebildet ist.

4. Fallsicherungsvorrichtung (20) nach einem der vorhergehenden Ansprüche, wobei das erste Blockierelement (28) fest mit dem Sicherungsteil (22) verbunden ist und/oder einteilig mit diesem ausgebildet ist.

5. Fallsicherungsvorrichtung (20) nach einem der vorhergehenden Ansprüche, wobei das zweite Blockierelement (30) dazu ausgebildet ist, erst nach dem Durchführen des Sicherungsteils (22) durch die beiden Durchgangsöffnungen (26, 28) an dem Sicherungsteil (22) befestigt zu werden.

6. Fallsicherungsvorrichtung (20) nach Anspruch 4 oder 5, wobei das erste Blockierelement (28) fest mit dem Sicherungsteil (22) verbunden ist und/oder einteilig mit diesem ausgebildet ist und wobei das zweite Blockierelement (30) dazu ausgebildet ist, erst nach dem Durchführen des Sicherungsteils (22) durch die beiden Durchgangsöffnungen (26, 28) an dem Sicherungsteil (22) befestigt zu werden.

7. Fallsicherungsvorrichtung (20) nach einem der vorhergehenden Ansprüche, wobei die jeweiligen Blockierelemente (28, 30) so gestaltet sind, dass diese nicht durch die jeweilige zugeordnete Durchgangsöffnung (24, 26) gleiten können.

8. Fallsicherungsvorrichtung (20) nach einem der vorhergehenden Ansprüche, wobei wenigstens eine der Durchgangsöffnungen (24) vom Innenraum (18) nach außen führt und wenigstens eines der beiden Blockierelemente (28, 30) außerhalb des Innenraums (18) angeordnet ist.

9. Fallsicherungsvorrichtung (20) nach nach einem der vorhergehenden Ansprüche, wobei der Innenraum (18) von einer Verschlusskappe verschlossen ist.

10. Fallsicherungsvorrichtung (20) nach einem der Ansprüche 8 oder 9, wobei wenigstens ein Mittenbereich des Sicherungsteils (22) innerhalb des Innenraums (18) angeordnet ist.

11. Fallsicherungsvorrichtung (20) nach einem der vorhergehenden Ansprüche, wobei die Fallsicherungsvorrichtung (20) so ausgebildet ist, dass das Sicherungsteil (22) bei Gebrauch des Tragsystems (10), insbesondere vor einem Bruch der kritischen Stelle (12), lastfrei ist.

12. Fallsicherungsvorrichtung (20) nach einem der vorhergehenden Ansprüche, wobei das Gelenk (12) der Tragvorrichtung als Gußteil aus Aluminium oder Stahl ausgebildet ist.

13. Tragsystem (10) zum vorzugsweise bewegbaren Halten wenigstens eines medizinischen Geräts mit wenigstens einer Fallsicherungsvorrichtung (20) nach einem der vorhergehenden Ansprüche.

14. Tragsystem (10) nach Anspruch 13, wobei das erste Tragelement (14), insbesondere ein erster Tragarm, und das zweite Tragelement (16), insbesondere ein zweiter Tragarm, , mittels eines Gelenks (12) beweglich miteinander verbunden sind.

15. Verfahren zur Anbringung einer Fallsicherungsvorrichtung (20) an einem Tragsystem (10) zum vorzugsweise bewegbaren Halten wenigstens eines medizinischen Geräts, mit wenigstens den folgenden Schritten:
- Bereitstellen wenigstens eines länglichen Sicherungsteils (22) mit einem an einem ersten Ende befestigten ersten Blockierelement (28) und einem zweiten, dem ersten Ende gegenüberliegenden freien Ende;
- Bereitstellen wenigstens einer Tragvorrichtung, welche wenigstens eine kritische Stelle (12), eine erste Durchgangsöffnung (24) in einem ersten von der kritischen Stelle (12) beabstandeten Bereich (14), und eine zweite Durchgangsöffnung (26) in einem zweiten, dem ersten Bereich (14) bezüglich der kritischen Stelle (12) gegenüberliegend beabstandeten Bereich (16), aufweist;
- Durchführen des freien Endes des Sicherungsteils (22) durch die beiden Durchgangsöffnungen (24, 26); und
- Befestigen eines zweiten Blockierelements (30) an dem freien Ende, so dass ein Herausgleiten des Sicherungsteils (22) aus den beiden Durchgangsöffnungen (24, 26) blockiert ist,
wobei die erste Durchgangsöffnung (24) an einem ersten Tragelement (14) und die zweite Durchgangsöffnung (26) an einem zweiten Tragelement (16) vorgesehen sind und wobei die Durchgangsöffnungen (24, 26) in einer einen Innenraum (18) begrenzenden Wandung der Tragvorrichtung vorgesehen sind, und wobei das Sicherungsteil (22) zwischen den Durchgangsöffnungen (24, 26) und an einer Ausnehmung (32) des ersten Tragelements (14) in den Innenraum (18) geführt wird.

16. Verfahren nach Anspruch 15, wobei das zweite Blockierelement (30) als Hülse ausgebildet ist, welche zum Befestigen an dem freien Ende auf das Sicherungsteil (22) aufgesteckt und mit diesem verpresst wird.

## Claims

1. Fall arrester device (20) for a carrier system (10) for preferably movably holding at least one medical device,
having at least one carrier device which comprises at least one critical point (12), and
having at least one elongate securing part (22),
wherein the carrier device comprises a first through-opening (24) in a first region (14) which is spaced apart from the critical point (12), and comprises a second through-opening (26) in a second region (16) which is opposite to the first region with respect to the critical point (12) and is spaced apart from the critical point, wherein the carrier system (10) has a first carrier element (14) which has a first through-opening (24) and a second carrier element (16) which has a second through-opening (26),
wherein the securing part (22) is routed through the two through-openings (24, 26), and the fall arrester device (20) comprises a first blocking element (28) which is fastened to a first end of the securing part (22), and a second blocking element (30) which fastened to a second end of the securing part (22) which is opposite to the first end thereof,
wherein the blocking elements (28, 30) are arranged in such a way that a sliding of the securing part (22) out of the two through-openings (24, 26) is blocked and **characterized in that**
the through-openings (24, 26) are provided in a wall delimiting an interior space (18) of the carrier device, wherein the securing part (22) is routed between the through-openings (24, 26) and a recess (32) of the first carrier element (14) into the interior space (18).

2. Fall arrester device (20) according to claim 1, wherein
the critical point (12) is a joint (12) of the carrier device, a bearing between two parts of the carrier device, where they are movably connected to each other, an angular piece of the carrier device, a junction, in particular a weld, of the carrier device, and/or a region of the carrier device having the weakest structure.

3. Fall arrester device (20) according to claim 1 or 2, wherein the elongate securing part (22) is configured as flexible element.

4. Fall arrester device (20) according to one of the preceding claims, wherein the first blocking element (28) is fixedly coupled to the securing part (22) and/or is formed integrally therewith as one part.

5. Fall arrester device (20) according to one of the preceding claims, wherein the second blocking element (30) is configured to be only fastened to the securing part (22) after the securing part (22) is routed through the two through-openings (26, 28).

6. Fall arrester device (20) according to claim 4 or 5, wherein the first blocking element (28) is fixedly connected with the securing part (22) and/or formed as a single piece therewith, and wherein the second blocking element (30) is configured to be fixed on the securing part (22) after routing the securing part (22) through the through-openings (24, 26) and a recess (32) of the first carrier element (14) into the interior space (18).

7. Fall arrester device (20) according to one of the preceding claims, wherein the respective blocking elements (28, 30) are configured in a way not to be able to slide through the respective associated through-opening (24, 26).

8. Fall arrester device (20) according to one of the preceding claims, wherein the carrier device delimits an interior space (18) and at least one of the through-openings (24) extends from the interior space (18) towards the outside and at least one of the two blocking elements (28, 30) is arranged outside of the interior space (18).

9. Fall arrester device (20) according to one of the preceding claims; wherein the interior space (18) is closed by a closure cap.

10. Fall arrester device (20) according to one of the claims 8 or 9, wherein at least one central region of the securing part (22) is arranged within the interior space (18).

11. Fall arrester device (20) according to one of the preceding claims, wherein the fall arrester device (20) is configured in a way that the securing part (22) is load-free during usage of the carrier system (10), in particular before a break occurs at the critical point (12).

12. Fall arrester device (20) according to one of the preceding claims, wherein the joint (12) of the carrier device is formed as a cast component made of aluminum or steel.

13. Carrier system (10) for preferably movably holding at least one medical device having at least one fall arrester device (20) according to one of the preceding claims.

14. Carrier system (10) according to claim 13, wherein the carrier system (10) comprises the first carrier element (14), in particular a first carrier arm, and a second carrier arm (16), in particular a second carrier arm, which are moveably coupled to each other by means of a joint (12).

15. Method for attaching a fall arrester device (20) to a carrier system (10) for preferably movably holding at least one medical device comprising at least the following steps:
- providing at least one elongate securing part (22) having one first blocking element (28) which is fastened to a first end, and a second free end opposite to the first end;
- providing at least one carrier device, which comprises at least one critical point (12), a first through-opening (24) in a first region (14) which is spaced apart from the critical point (12), and a second through-opening (26) in a second region (16) which is opposite to the first region (14) with respect to the critical point (12) and is spaced apart from the critical point;
- routing the free end of the said securing part (22) through the two through-openings (24, 26); and
- fastening of a second blocking element (30) to the free end, in such a way that a sliding of the securing part (22) out of the two through-openings (24, 26) is blocked,
wherein the first through-opening (24) is provided on a first carrier element (14) and the second through-opening on a second carrier element (16) and the through-openings (24, 26) are provided in a wall delimiting an interior space (18) of the carrier device, wherein the securing part (22) is routed between the through-openings (24, 26) and a recess (32) of the first carrier element (14) into the interior space (18).

16. Method according to claim 15, wherein the second blocking element (30) is configured as sleeve which is fitted on the free end on the securing part (22) for fastening and is grouted therewith.

## Revendications

1. Dispositif de sécurité antichute (20) pour un système de support (10) pour retenir de préférence de manière déplaçable au moins un appareil médical,
avec au moins un dispositif de support, lequel présente au moins un point critique (12), et
avec au moins une partie de fixation allongée (22),
dans lequel le dispositif de support présente une première ouverture de passage (24) dans une première zone (14) espacée du point critique (12) et une deuxième ouverture de passage (26) dans une deuxième zone (16) espacée du point critique, opposée à la première zone par rapport au point critique (12), dans lequel le système de support (10) présente un premier élément de support (14), lequel présente la première ouverture de passage (24), et un deuxième élément de support (16), lequel présente la deuxième ouverture de passage (26),
dans lequel la partie de fixation (22) est guidée à travers les deux ouvertures de passage (24, 26) et le dispositif de sécurité antichute (20) présente un premier élément de blocage (28) fixé à une première extrémité de la partie de fixation (22) et un deuxième élément de blocage (30) fixé à une deuxième extrémité, opposée à la première extrémité, de la partie de fixation (22),
dans lequel les éléments de blocage (28, 30) sont disposés de manière telle qu'un glissement de la partie de fixation (22) hors des deux ouvertures de passage (24, 26) est entravé, et
**caractérisé en ce que** les ouvertures de passage (24, 26) sont prévues dans une paroi délimitant un espace interne (18) du dispositif de support, dans lequel la partie de fixation (22) est guidée entre les ouvertures de passage (24, 26) et sur un évidement (32) du premier élément de support (14) dans l'espace interne (18).

2. Dispositif de sécurité antichute (20) selon la revendication 1, dans lequel le point critique (12) est une articulation (12) du dispositif de support, un logement entre deux parties du dispositif de support, sur lequel celles-ci sont reliées ensemble de façon mobile, un raccord angulaire du dispositif de support, un point de jonction, plus particulièrement une soudure, du dispositif de support, et/ou une zone structurellement plus faible du dispositif de support.

3. Dispositif de sécurité antichute (20) selon la revendication 1 ou 2, dans lequel la partie de fixation allongée (22) est conçue comme un élément mou à la flexion.

4. Dispositif de sécurité antichute (20) selon l'une des revendications précédentes, dans lequel le premier élément de blocage (28) est relié fixement à la partie de fixation (22) et/ou est conçu d'une seule pièce avec celle-ci.

5. Dispositif de sécurité antichute (20) selon l'une des revendications précédentes, dans lequel le deuxième élément de blocage (30) est conçu pour être fixé à la partie de fixation (22) uniquement après le passage de la partie de fixation (22) à travers les deux ouvertures de passage (26, 28).

6. Dispositif de sécurité antichute (20) selon la revendication 4 ou 5, dans lequel le premier élément de blocage (28) est relié fixement à la partie de fixation (22) et/ou est conçu d'une seule pièce avec celle-ci et dans lequel le deuxième élément de blocage (30) est conçu pour être fixé à la partie de fixation (22) uniquement après le passage de la partie de fixation (22) à travers les deux ouvertures de passage (26, 28).

7. Dispositif de sécurité antichute (20) selon l'une des revendications précédentes, dans lequel les éléments de blocage respectifs (28, 30) sont configurés d'une manière telle que ceux-ci ne peuvent pas glisser à travers l'ouverture de passage affectée respective (24, 26).

8. Dispositif de sécurité antichute (20) selon l'une des revendications précédentes, dans lequel au moins l'une des ouvertures de passage (24) conduit de l'espace interne (18) vers l'extérieur et au moins l'un des deux éléments de blocage (28, 30) est disposé à l'extérieur de l'espace interne (18).

9. Dispositif de sécurité antichute (20) selon l'une des revendications précédentes, dans lequel l'espace interne (18) est fermé par un bouchon de fermeture.

10. Dispositif de sécurité antichute (20) selon l'une des revendications 8 ou 9, dans lequel au moins une zone centrale de la partie de fixation (22) est disposée à l'intérieur de l'espace interne (18).

11. Dispositif de sécurité antichute (20) selon l'une des revendications précédentes, dans lequel le dispositif de sécurité antichute (20) est conçu de sorte que la partie de fixation (22) est sans charge lors de l'utilisation du système de transport (10), plus particulièrement avant une rupture du point critique (12).

12. Dispositif de sécurité antichute (20) selon l'une des revendications précédentes, dans lequel l'articulation (12) du dispositif de support est conçue comme une pièce coulée en aluminium ou acier.

13. Système de support (10) pour retenir de préférence de manière déplaçable au moins un appareil médical avec au moins un dispositif de support antichute (20) selon l'une des revendications précédentes.

14. Système de support (10) selon la revendication 13, dans lequel le premier élément de support (14), plus particulièrement un premier bras de support, et le deuxième élément de support (16), plus particulièrement un deuxième bras de support, sont reliés ensemble de façon mobile au moyen d'une articulation (12).

15. Procédé de montage d'un dispositif de sécurité antichute (20) sur un système de support (10) pour retenir de préférence de manière déplaçable au moins un appareil médical, comportant au moins les étapes suivantes :
- mise à disposition d'au moins une partie de fixation allongée (22) avec un premier élément de blocage (28) fixé à une première extrémité et une deuxième extrémité libre opposée à la première extrémité ;
- mise à disposition d'au moins un dispositif de support, lequel présente au moins un point critique (12), une première ouverture de passage (24) dans une première zone (14) espacée du point critique (12), et une deuxième ouverture de passage (26) dans une deuxième zone (16) espacée se trouvant à l'opposé de la première zone (14) par rapport au point critique (12) ;
- passage de l'extrémité libre de la partie de fixation (22) à travers les deux ouvertures de passage (24, 26) ; et
- fixation d'un deuxième élément de blocage (30) à l'extrémité libre de sorte qu'un glissement de la partie de fixation (22) hors des deux ouvertures de passage (24, 26) est entravé,
dans lequel la première ouverture de passage (24) est prévue sur un premier élément de support (14) et la deuxième ouverture de passage (26) sur un deuxième élément de support (16) et dans lequel les ouvertures de passage (24, 26) sont prévues dans une paroi délimitant un espace interne (18) du dispositif de support, et dans lequel la partie de fixation (22) est guidée entre les ouvertures de passage (24, 26) et sur un évidement (32) du premier élément de support (14) dans l'espace interne (18).

16. Procédé selon la revendication 15, dans lequel le deuxième élément de blocage (30) est conçu comme un manchon, lequel pour la fixation à l'extrémité libre est inséré sur la partie de fixation (22) et est comprimé avec celle-ci.
